# EUROPEAN PATENT APPLICATION

(11) **EP 4 040 311 A1**
(43) Date of publication of application: **10.08.2022**
(21) Application number: 22151818.6
(22) Date of filing: 17.01.2022
(51) Int. Cl.: G06F 16/84, G06F 16/906, G06N 20/00, G06Q 10/00, G16H 10/60

(54) **UTILIZING MACHINE LEARNING AND NATURAL LANGUAGE PROCESSING TO EXTRACT AND VERIFY VACCINATION DATA**

(30) Priority: 03.02.2021 US 202163199920 P; 15.03.2021 US 202117249819
(71) Applicant: Accenture Global Solutions Limited, Dublin 4 (IE)
(72) Inventor: PRIESTAS, James Robert, Alexandria, 22314 (US); O'GARA, Tara Lynn, Chicago, 60611 (US); SIMANEK, Michael Edward, Chicago, 60610 (US); DONKER, Matthijs Radboud, Colorado Springs, 80904 (US); ROBINSON, Michael Jesse, Washington, 20017 (US)
(74) Representative: Müller-Boré & Partner Patentanwälte PartG mbB

(57) **Abstract**

A device may receive, based on a request, document data identifying structured and unstructured documents associated with vaccinations received by users and may perform natural language processing on the document data to generate processed document data. The device may process the processed document data, with a machine learning model, to extract vaccination data from the processed document data and may transcribe the vaccination data into corresponding fields of a data structure. The device may receive, from a user device, a request for vaccination data associated with a user and may retrieve the vaccination data from the corresponding fields of the data structure based on the request. The device may provide the vaccination data, to the user device, to enable verification of the vaccination data.

## Description

### CROSS-REFERENCE TO RELATED APPLICATION

This Patent Application claims priority to U.S. Provisional Patent Application No. 63/199,920, filed on February 3, 2021, and entitled "UTILIZING MACHINE LEARNING AND NATURAL LANGUAGE PROCESSING TO EXTRACT AND VERIFY VACCINATION DATA." The disclosure of the prior Application is considered part of and is incorporated by reference into this Patent Application.

### BACKGROUND

Forms or documents of various types are widely used for collecting information for coronavirus disease (COVID) purposes. Medical, commercial, educational, and governmental organizations use COVID documents of various formats (e.g., formats associated with the Centers for Disease Control (CDC) COVID vaccination record card, other COVID vaccination forms of the United States and other countries, attestation of COVID vaccine forms, COVID antigen/antibody laboratory tests, and COVID forms) for collecting information and for record keeping purposes associated with COVID.

### SUMMARY

In some implementations, a method may include receiving document data identifying structured and unstructured documents associated with vaccinations received by users and performing natural language processing on the document data to generate processed document data. The method may include processing the processed document data, with a machine learning model, to extract vaccination data from the processed document data and transcribing the vaccination data into corresponding fields of a data structure. The method may include receiving, from a user device associated with an authority agent, a particular request for particular vaccination data associated with a user of the users and retrieving the particular vaccination data from the corresponding fields of the data structure based on the particular request. The method may include providing the particular vaccination data, to the user device associated with the authority agent, to enable verification of the particular vaccination data.

In some implementations, a device includes one or more memories and one or more processors to train a machine learning model with historical document data identifying historical structured and unstructured documents associated with historical vaccinations and provide a request for document data. The one or more processors may receive, based on the request, document data identifying structured and unstructured documents associated with vaccinations received by users and may perform natural language processing on the document data to generate processed document data. The one or more processors may process the processed document data, with the machine learning model, to extract vaccination data from the processed document data and may assign the vaccination data into corresponding fields of a data structure. The one or more processors may verify the vaccination data, from the corresponding fields, with a registration authority and may receive, from a user device associated with an authority agent, a particular request for particular vaccination data associated with a user of the users. The one or more processors may retrieve the particular vaccination data from the corresponding fields of the data structure based on the particular request and may provide the particular vaccination data, to the user device associated with the authority agent, to enable verification of the particular vaccination data.

In some implementations, a non-transitory computer-readable medium may store a set of instructions that includes one or more instructions that, when executed by one or more processors of a device, cause the device to provide a request for document data and receive, based on the request, document data identifying structured and unstructured documents associated with vaccinations received by users. The one or more instructions may cause the device to perform natural language processing on the document data to generate processed document data and process the processed document data, with a machine learning model, to extract vaccination data from the processed document data. The one or more instructions may cause the device to transcribe the vaccination data into corresponding fields of a data structure and verify the vaccination data, from the corresponding fields, with a registration authority.

### BRIEF DESCRIPTION OF THE DRAWINGS

Figs. 1A-1E are diagrams of an example implementation described herein.
Fig. 2 is a diagram illustrating an example of training and using a machine learning model in connection with extracting and verifying vaccination data.
Fig. 3 is a diagram of an example environment in which systems and/or methods described herein may be implemented.
Fig. 4 is a diagram of example components of one or more devices of Fig. 3.
Fig. 5 is a flowchart of an example process for utilizing machine learning and natural language processing to extract and verify vaccination data.

### DETAILED DESCRIPTION

The following detailed description of example implementations refers to the accompanying drawings. The same reference numbers in different drawings may identify the same or similar elements.

The advent of computers and communication networks resulted in documents being completed online so that people no longer have to fill out paper forms. In addition, digitized records, including electronic and scanned copies of paper documents, are now generated using computers. These electronic documents are shared over the communication networks to save time and resources that may be otherwise required for generating and exchanging paper documents. These documents may contain data in structured and unstructured formats. A structured document may include embedded code which enables arranging information in a specified format. Unstructured documents include free form arrangements, wherein the structure, style, and content of information in the original documents may not be preserved. Many entities create and store large unstructured electronic documents that may include content from multiple sources.

Due to recent CDC guidelines and government regulations, various systems have attempted to utilize information from medical documents to perform operations in expedited timeframes. It is relatively easy to programmatically extract information from structured documents that have a well-defined format, such as extracting data from fields in a form where the fields are at known locations in the form (e.g., data in a tabular arrangement). However, when the documents include large unstructured documents, it is technically difficult to extract information that may be needed to perform operations with systems. Unstructured documents often do not have well-defined formats, making it difficult to programmatically parse and extract information from such documents. Many of the documents are handwritten, which makes it even more difficult to automatically extract information.

Thus, current techniques for performing operations with unstructured documents waste computing resources (e.g., processing resources, memory resources, communication resources, and/or the like), networking resources, human resources, and/or the like associated with incorrectly extracting information from unstructured documents, making poor decisions based on the incorrect information, performing incorrect operations based on the incorrect information, and/or the like.

Some implementations described herein relate to a verification system that utilizes machine learning and natural language processing to extract and verify vaccination data. For example, the verification system may receive, based on a request, document data identifying structured and unstructured documents associated with vaccinations received by users and may perform natural language processing on the document data to generate processed document data. The verification system may process the processed document data, with a machine learning model, to extract vaccination data from the processed document data and may transcribe the vaccination data into corresponding fields of a data structure. The verification system may receive, from a user device associated with an authority agent, a particular request for particular vaccination data associated with a user of the users and may retrieve the particular vaccination data from the corresponding fields of the data structure based on the particular request. The verification system may provide the particular vaccination data, to the user device associated with the authority agent, to enable verification of the particular vaccination data.

In this way, the verification system utilizes machine learning and natural language processing to extract and verify vaccination data. The verification system may process electronic documents, such as structured and unstructured documents, to extract required information and enable automatic execution of processes based on the extracted information. The verification system may utilize the extracted information to build internal master documents that enable generation of forms, contracts, and/or the like during the automatic execution of the processes. This, in turn, conserves computing resources, networking resources, human resources, and/or the like that would otherwise have been wasted in incorrectly extracting information from unstructured documents, making poor decisions based on the incorrect information, performing incorrect operations based on the incorrect information, and/or the like.

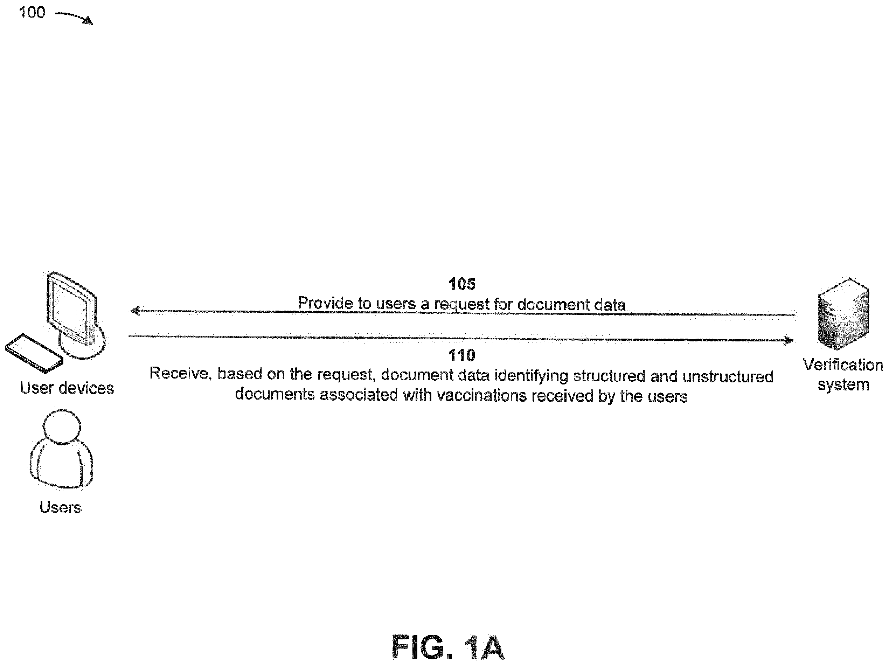

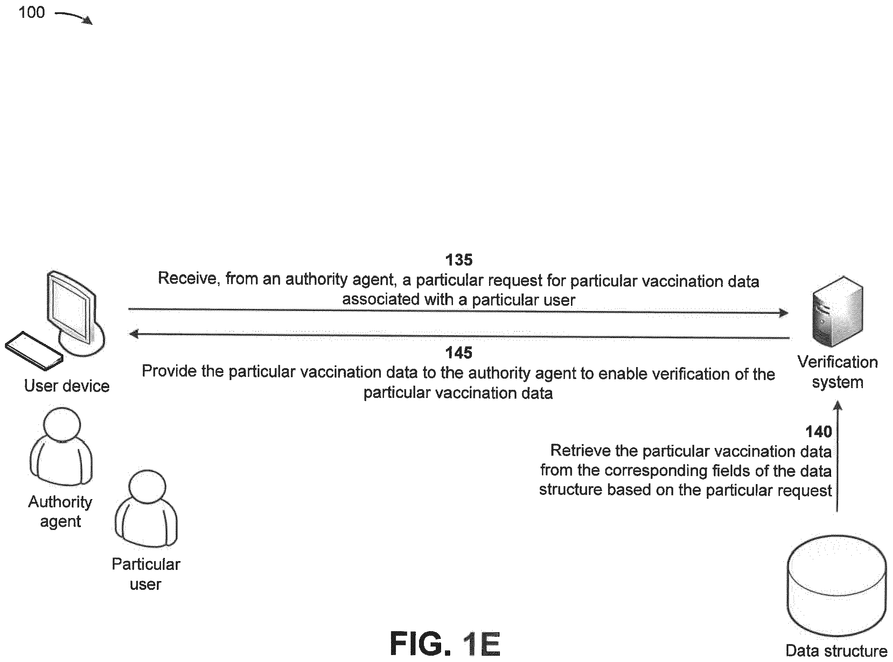

Figs. 1A-1E are diagrams of an example 100 associated with utilizing machine learning and natural language processing to extract and verify vaccination data. As shown in Figs. 1A-1E, example 100 includes user devices associated with users and a verification system. The user devices and the verification system are described in greater detail below.

As shown in Fig. 1A, and by reference number 105, the verification system may provide to users a request for document data. For example, the verification system may provide the request for document data to user devices associated with the users. In some implementations, the user devices may include applications that cause the user devices to provide the document data to the verification system automatically or periodically. In such implementations, the verification system need not generate and provide the request for the document data to the user devices.

As further shown in Fig. 1A, and by reference number 110, the verification system may receive, based on the request, document data identifying structured and unstructured documents associated with vaccinations received by the users. For example, the user devices may generate the document data based on the request and may provide the document data to the verification system. The verification system may receive the document data from the user devices. In some implementations, the user devices may include applications that cause the user devices to provide the document data to the verification system automatically or periodically. In some implementations, the verification system may provide the request to and receive the document data from devices other than the user devices, such as from one or more server devices, from a cloud computing environment, and/or the like.

The document data may identify structured and unstructured documents that include patient names (e.g., usernames), COVID test results, pharmaceutical drug company names, specimen numbers, vaccine lot numbers, clinic site information, and/or the like. Documents of various types may be used for collecting information for COVID purposes. Medical, commercial, educational, and governmental organizations use COVID documents of various formats, such as a CDC COVID vaccination record card, other COVID vaccination forms, attestation of COVID vaccination forms, COVID antigen/antibody laboratory tests, COVID forms for collecting information associated with interactions with COVID, and/or the like. The structured documents may include embedded code which enables arranging information in specified formats. For example, the embedded code may define the specified formats and how to arrange information in the specified formats for the structured documents. The unstructured documents may include free form arrangements (e.g., a plurality of formats), wherein structures, styles, and content of information in original documents may not be preserved in the unstructured documents. Some entities may create and store large quantities of unstructured documents that may include content from multiple sources.

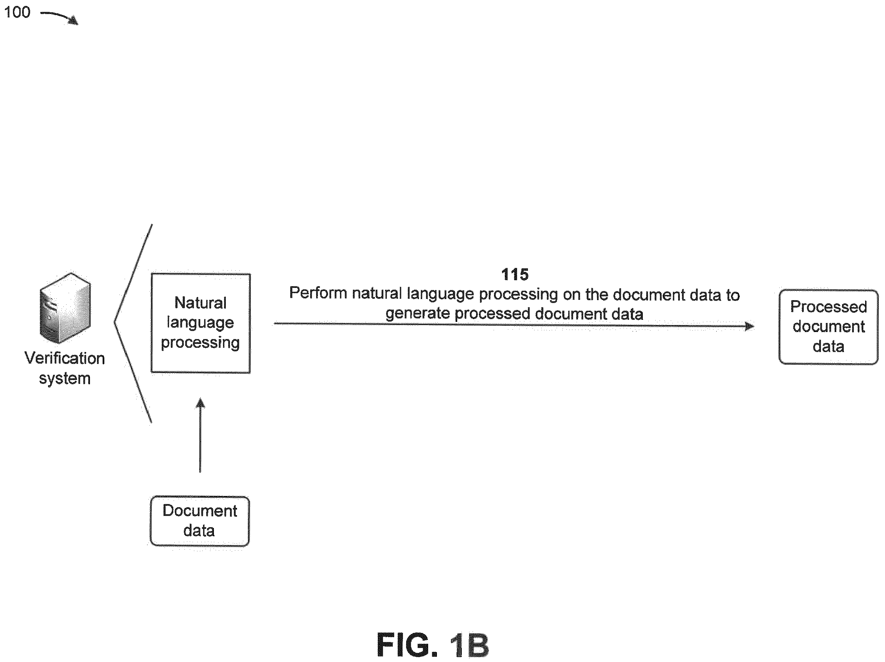

As shown in Fig. 1B, and by reference number 115, the verification system may perform natural language processing on the document data to generate processed document data. For example, the verification system may perform natural language processing on the document data to decipher textual information (e.g., handwritten text, textual fields provided in tables, text provided in graphs, and/or the like) provided in the document data. The textual information may indicate whether each of the users received one vaccination for COVID, received two vaccines for COVID, tested negative for COVID, filled out a form verifying no exposure to COVID, and/or the like.

In some implementations, prior to performing the natural language processing, the verification system may convert documents of different formats (e.g., from the document data) into homogenous documents (e.g., with a common format) via a computer vision model, optical character recognition (OCR), and/or the like. By converting the document data into homogeneous documents, the verification system may improve precision of the processed document data generated by the natural language processing, may improve automatic resolution of discrepancies (e.g., differences) in the processed document data by a machine learning model (e.g., as described below), and may improve generation of a master data structure that includes vaccination data. The structured and unstructured documents of the document data may include different formats (e.g., heterogeneous data), such as typed textual data, handwritten text, data presented as tables, graphs, and other non-textual formats, and/or the like. The verification system may analyze such heterogeneous data, with varying formats, to identify and compare information presented in the heterogeneous data. In this way, the verification system may improve a speed and an accuracy of the natural language processing and the machine learning model, which may conserve computing resources, networking resources, and/or the like. The verification system may also enable external computing systems to consume data directly as homogenous documents as opposed to extracting data from heterogenous documents of different data formats.

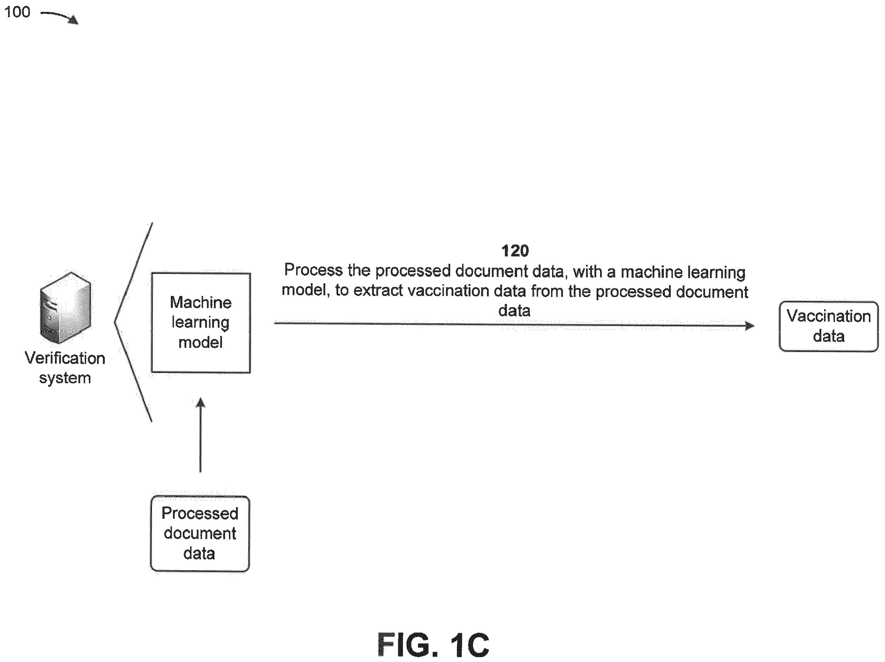

As shown in Fig. 1C, and by reference number 120, the verification system may process the processed document data, with a machine learning model, to extract vaccination data from the processed document data. For example, the machine learning model may extract usernames, COVID test results, pharmaceutical company names, specimen numbers, vaccine lot numbers, clinic site information, and/or the like from the processed document data. In some implementations, the machine learning model is a classifier model that classifies the processed document data into categories that may be used to verify the processed document data against a registry or some other database.

The machine learning model may include a machine learning-based domain model that includes domain-specific terminology, definitions of industry terms, and/or possible fields of various data types that may be included in the documents of the document data. Accordingly, the machine learning model may utilize such information to identify vaccination data within the documents (e.g., patient names, COVID test results, pharmaceutical companies, specimen numbers, vaccine lot numbers, clinic sites, and/or the like). The verification system may identify an intent based on the documents included in the document data and may select the machine learning model from a plurality of machine learning-based domain models based on the intent. The intent may include an identifier or another indicator of a domain associated with the document data. Accordingly, different vaccination data may be extracted based on the machine learning-based domain model selected by the verification system.

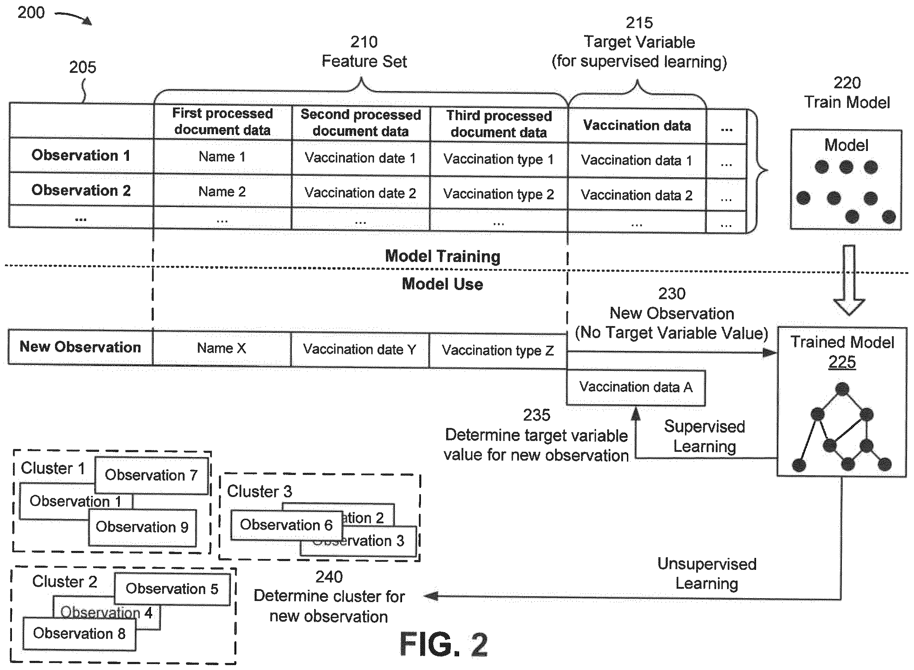

In some implementations, the machine learning model may identify one or more discrepancies (e.g., differences) in the processed document data and may determine one or more solutions to the one or more discrepancies. Alternatively, or additionally, the machine learning model may receive feedback associated with the one or more discrepancies. The machine learning model may extract the vaccination data from the processed document data based on the one or more solutions and/or the feedback. Prior to receiving the document data, the machine learning model may be trained with historical document data identifying historical structured and unstructured documents associated with historical vaccinations, as described below in connection with Fig. 2.

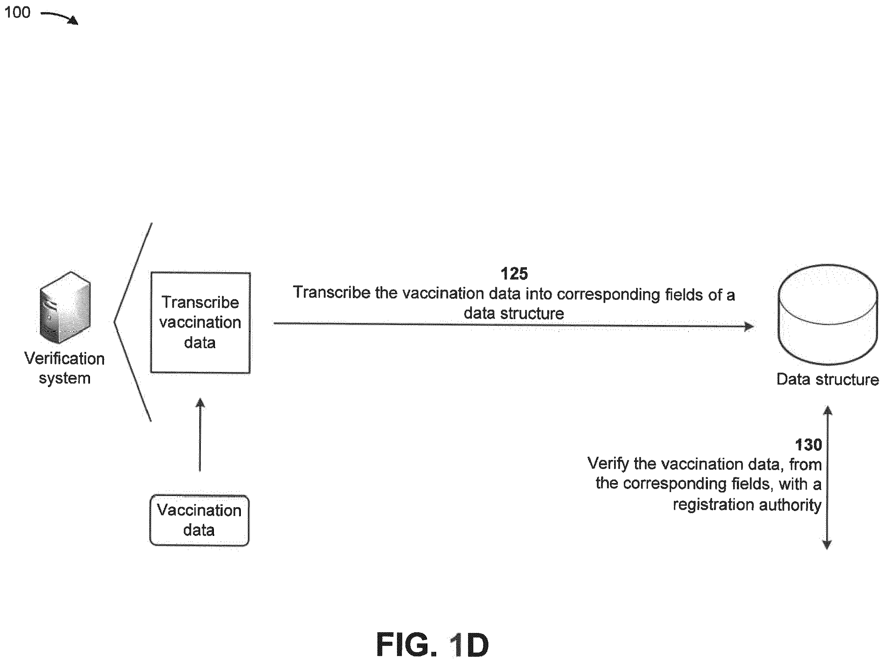

As shown in Fig. 1D, and by reference number 125, the verification system may transcribe the vaccination data into corresponding fields of a data structure. For example, the data structure may include fields for patient name, COVID test results, pharmaceutical company name, specimen number, vaccine lot number, clinic site, and/or the like, and the verification system may transcribe or assign the vaccination data to such fields. The data structure may enable external computing systems to consume the vaccination data directly as homogenous , as opposed to extracting vaccination data from heterogenous documents of different data formats. The data structure may provide a master repository for the vaccination data and may enable the vaccination data to be quickly and easily located and retrieved by external computing systems.

As further shown in Fig. ID, and by reference number 130, the verification system may verify the vaccination data, from the corresponding fields, with a registration authority. For example, the verification system may verify the vaccination data with a state vaccination registry, a national vaccination registry, an international vaccination registry, and/or the like. The verification system may request (e.g., from a server device associated with a registration authority) vaccination data that corresponds to vaccination data stored in the data structure and may receive the corresponding vaccination data. The verification system may compare the corresponding vaccination data with the vaccination data in the data structure to verify whether the corresponding vaccination data matches the vaccination data in the data structure. Alternatively, the verification may provide the vaccination data (e.g., to the server device associated with the registration authority) and may request that the server device verify whether the corresponding vaccination data matches the vaccination data. If any of the vaccination data is not verified, the verification system may request (e.g., from the user devices) that such unverified data be corrected or updated so that such unverified data may be verified with the registration authority. In some implementations, the verification system may receive, from the registration authority, feedback identifying one or more discrepancies in the vaccination data. In such implementations, the verification system may correct the one or more discrepancies identified in the feedback to generate corrected vaccination data and may verify the corrected vaccination data with the registration authority.

As shown in Fig. IE, and by reference number 135, the verification system may receive, from an authority agent, a particular request for particular vaccination data associated with a particular user. For example, the verification system may receive the particular request from a user device controlled by and/or displayed to the authority agent (e.g., an airport security agent, a government agent, and/or the like). The particular request may seek to validate a vaccination by the particular user prior to allowing the particular user to perform an action (e.g., enter a country, board an airplane, board a train, and/or the like). The particular request may include a name of the particular user. The particular vaccination data may include data identifying the name of the particular user, a vaccination or vaccinations received by the particular user, a COVID test result of the particular user, a vaccine lot number associated with the particular user, and/or the like.

As further shown in Fig. IE, and by reference number 140, the verification system may retrieve the particular vaccination data from the corresponding fields of the data structure based on the particular request. For example, the verification system may utilize the name of the particular user to identify and retrieve the particular vaccination data from the corresponding fields of the data structure. The verification system may identify the name of the particular user from an entry included in the patient name field of the data structure. The verification system may retrieve the particular vaccination data from entries of other fields of the data structure that correspond to the entry included in the patient name field.

As further shown in Fig. IE, and by reference number 145, the verification system may provide the particular vaccination data and/or data verifying the particular vaccination data (e.g., "vaccination verified") to the authority agent to enable verification of the particular vaccination data. For example, the verification system may provide the particular vaccination data to the user device associated with the authority agent, and the authority agent may verify the particular user to perform an action (e.g., enter a country, board an airplane, board a train, and/or the like) based on the particular vaccination data (e.g., via the user comparing the particular vaccination data with verified vaccination data). Alternatively, the verification system may verify the particular user to perform the action based on the particular vaccination data retrieved from the data structure for the particular user. In such instances, the verification system may provide data verifying the particular vaccination data to the user device associated with the authority agent and the user device may display the data verifying the particular vaccination data to the authority agent. The authority agent may then allow the particular user to perform the action.

In some implementations, the verification system may receive, from the user device associated with the authority agent, an additional information request associated with the particular vaccination data and may identify additional information based on the additional information request. The verification system may provide the additional information, to the user device associated with the authority agent, to enable verification of the particular vaccination data.

In some implementations, the verification system may receive an update to the particular vaccination data associated with the user and may update the particular vaccination data in the data structure based on the update. The verification system may also retrain the machine learning model based on the update. The verification system may utilize the update as additional training data for retraining the machine learning model, thereby increasing the quantity of training data available for training the machine learning model. Accordingly, the verification system may conserve computing resources associated with identifying, obtaining, and/or generating historical data for training the machine learning model relative to other systems for identifying, obtaining, and/or generating historical data for training machine learning models.

In this way, the verification system utilizes machine learning and natural language processing to extract and verify vaccination data. The verification system may process electronic documents, such as structured and unstructured documents, to extract required information and enable automatic execution of processes based on the extracted information. The verification system may utilize the extracted information to build internal master documents that enable generation of forms, contracts, and/or the like during the automatic execution of the processes. This, in turn, conserves computing resources, networking resources, human resources, and/or the like that would otherwise have been wasted in incorrectly extracting information from unstructured documents, making poor decisions based on the incorrect information, performing incorrect operations based on the incorrect information, and/or the like.

The verification system may employ a machine learning-based domain model that includes domain-specific terminology, definitions of industry terms, and/or possible fields of various data types that may be included in documents received for processing by the verification system. Accordingly, automatic execution of processes from various domains, that require the identification of specific key-value pairs within a document (e.g., a patient name, COVID test result, a pharmaceutical company, a specimen number, a vaccine lot number, a clinic site, and/or the like), may be provided based on a particular domain model employed by the verification system. An intent may be identified, by the verification system, from a request that includes one or more documents. The intent may be an identifier or other indicator of an automatically executed process that the verification system enables in response to receiving the request (e.g., automatically receiving the one or more documents). The intent may be further processed via employing the domain model and one or more other data sources, including external knowledge bases. Based on the identified intent, a document may be processed via one or more different process streams (e.g., via one or more different domain models, one or more different knowledge bases, one or more different data sources, and/or the like). Accordingly, different input fields may be extracted and identified using the domain model and different internal master documents may be created based on a selected process stream. For example, the domain model may identify the different input fields from the different internal master documents, and may extract the different input fields from the identified fields in the different internal master documents. Correspondingly, discrepancy resolutions and user interfaces employed to present information from the verification system may also differ based on the process streams.

The verification system may effectively convert documents of different formats into homogeneous documents via computer vision or optical character recognition, which may improve the precision of information that is extracted from the documents and compared. The verification system may automatically resolve discrepancies using the machine learning model and may automatically execute downstream processes, such as creating internal master documents. The documents processed by the verification system may include structured and unstructured documents of different formats, such as typed textual data, handwritten text, tables, graphs, or other non-textual formats. The verification system may analyze such heterogeneous documents with varying formats to identify and compare information presented therein. The data transformations from other formats to textual data types using computer vision, optical character recognition, and/or a machine learning model provide dynamic presentation of the data from non-editable image files and enable robotic process automation via creation of internal master documents from the extracted and processed data. Automating downstream processes improves the speed and accuracy of not only the verification system (e.g., which may implement such automated processes) but also of other external computing systems that may consume data directly as homogeneous internal master documents rather than extracting data from nonhomogeneous data sources. The verification system may utilize computer vision to extract specific data elements and may perform a validation process by comparing extracted data to a validated data source (such as, for example, a state vaccination registry). For example, the verification system may utilize Fast Healthcare Interoperability Resources (FHIR) to communicate with state and/or country vaccine registries to validate vaccinations and/or test result data.

As indicated above, Figs. 1A-1E are provided as an example. Other examples may differ from what is described with regard to Figs. 1A-1E. The number and arrangement of devices shown in Figs. 1A-1E are provided as an example. In practice, there may be additional devices, fewer devices, different devices, or differently arranged devices than those shown in Figs. 1A-1E. Furthermore, two or more devices shown in Figs. 1A-1E may be implemented within a single device, or a single device shown in Figs. 1A-1E may be implemented as multiple, distributed devices. Additionally, or alternatively, a set of devices (e.g., one or more devices) shown in Figs. 1A-1E may perform one or more functions described as being performed by another set of devices shown in Figs. 1A-1E.

Fig. 2 is a diagram illustrating an example 200 of training and using a machine learning model in connection with extracting and verifying vaccination data. The machine learning model training and usage described herein may be performed using a machine learning system. The machine learning system may include or may be included in a computing device, a server, a cloud computing environment, and/or the like, such as the verification system described in more detail elsewhere herein.

As shown by reference number 205, a machine learning model may be trained using a set of observations. The set of observations may be obtained from historical data, such as data gathered during one or more processes described herein. In some implementations, the machine learning system may receive the set of observations (e.g., as input) from the verification system, as described elsewhere herein.

As shown by reference number 210, the set of observations includes a feature set. The feature set may include a set of variables, and a variable may be referred to as a feature. A specific observation may include a set of variable values (or feature values) corresponding to the set of variables. In some implementations, the machine learning system may determine variables for a set of observations and/or variable values for a specific observation based on input received from the verification system. For example, the machine learning system may identify a feature set (e.g., one or more features and/or feature values) by extracting the feature set from structured data, by performing natural language processing to extract the feature set from unstructured data, by receiving input from an operator, and/or the like.

As an example, a feature set for a set of observations may include a first feature of first processed document data, a second feature of second processed document data, a third feature of third processed document data, and so on. As shown, for a first observation, the first feature may have a value of name 1, the second feature may have a value of vaccination data 1, the third feature may have a value of vaccination type 1, and so on. These features and feature values are provided as examples and may differ in other examples.

As shown by reference number 215, the set of observations may be associated with a target variable. The target variable may represent a variable having a numeric value, may represent a variable having a numeric value that falls within a range of values or has some discrete possible values, may represent a variable that is selectable from one of multiple options (e.g., one of multiple classes, classifications, labels, and/or the like), may represent a variable having a Boolean value, and/or the like. A target variable may be associated with a target variable value, and a target variable value may be specific to an observation. In example 200, the target variable is vaccination data, which has a value of vaccination data 1 for the first observation.

The target variable may represent a value that a machine learning model is being trained to predict, and the feature set may represent the variables that are input to a trained machine learning model to predict a value for the target variable. The set of observations may include target variable values so that the machine learning model can be trained to recognize patterns in the feature set that lead to a target variable value. A machine learning model that is trained to predict a target variable value may be referred to as a supervised learning model.

In some implementations, the machine learning model may be trained on a set of observations that do not include a target variable. This may be referred to as an unsupervised learning model. In this case, the machine learning model may learn patterns from the set of observations without labeling or supervision, and may provide output that indicates such patterns, such as by using clustering and/or association to identify related groups of items within the set of observations.

As shown by reference number 220, the machine learning system may train a machine learning model using the set of observations and using one or more machine learning algorithms, such as a regression algorithm, a decision tree algorithm, a neural network algorithm, a k-nearest neighbor algorithm, a support vector machine algorithm, and/or the like. After training, the machine learning system may store the machine learning model as a trained machine learning model 225 to be used to analyze new observations.

As shown by reference number 230, the machine learning system may apply the trained machine learning model 225 to a new observation, such as by receiving a new observation and inputting the new observation to the trained machine learning model 225. As shown, the new observation may include a first feature of name X, a second feature of vaccination data Y, a third feature of vaccination type Z, and so on, as an example. The machine learning system may apply the trained machine learning model 225 to the new observation to generate an output (e.g., a result). The type of output may depend on the type of machine learning model and/or the type of machine learning task being performed. For example, the output may include a predicted value of a target variable, such as when supervised learning is employed. Additionally, or alternatively, the output may include information that identifies a cluster to which the new observation belongs, information that indicates a degree of similarity between the new observation and one or more other observations, and/or the like, such as when unsupervised learning is employed.

As an example, the trained machine learning model 225 may predict vaccination data A for the target variable of the cluster for the new observation, as shown by reference number 235. Based on this prediction, the machine learning system may provide a first recommendation, may provide output for determination of a first recommendation, may perform a first automated action, may cause a first automated action to be performed (e.g., by instructing another device to perform the automated action), and/or the like.

In some implementations, the trained machine learning model 225 may classify (e.g., cluster) the new observation in a cluster, as shown by reference number 240. The observations within a cluster may have a threshold degree of similarity. As an example, if the machine learning system classifies the new observation in a first cluster (e.g., a first processed document data cluster), then the machine learning system may provide a first recommendation. Additionally, or alternatively, the machine learning system may perform a first automated action and/or may cause a first automated action to be performed (e.g., by instructing another device to perform the automated action) based on classifying the new observation in the first cluster.

As another example, if the machine learning system were to classify the new observation in a second cluster (e.g., a second processed document data cluster), then the machine learning system may provide a second (e.g., different) recommendation and/or may perform or cause performance of a second (e.g., different) automated action.

In some implementations, the recommendation and/or the automated action associated with the new observation may be based on a target variable value having a particular label (e.g., classification, categorization, and/or the like), may be based on whether a target variable value satisfies one or more thresholds (e.g., whether the target variable value is greater than a threshold, is less than a threshold, is equal to a threshold, falls within a range of threshold values, and/or the like), may be based on a cluster in which the new observation is classified, and/or the like.

In this way, the machine learning system may apply a rigorous and automated process for extracting and verifying vaccination data. The machine learning system enables recognition and/or identification of tens, hundreds, thousands, or millions of features and/or feature values for tens, hundreds, thousands, or millions of observations, thereby increasing accuracy and consistency and reducing delay associated with extracting and verifying vaccination data relative to requiring computing resources to be allocated for tens, hundreds, or thousands of operators to manually extract and verify vaccination data.

As indicated above, Fig. 2 is provided as an example. Other examples may differ from what is described in connection with Fig. 2.

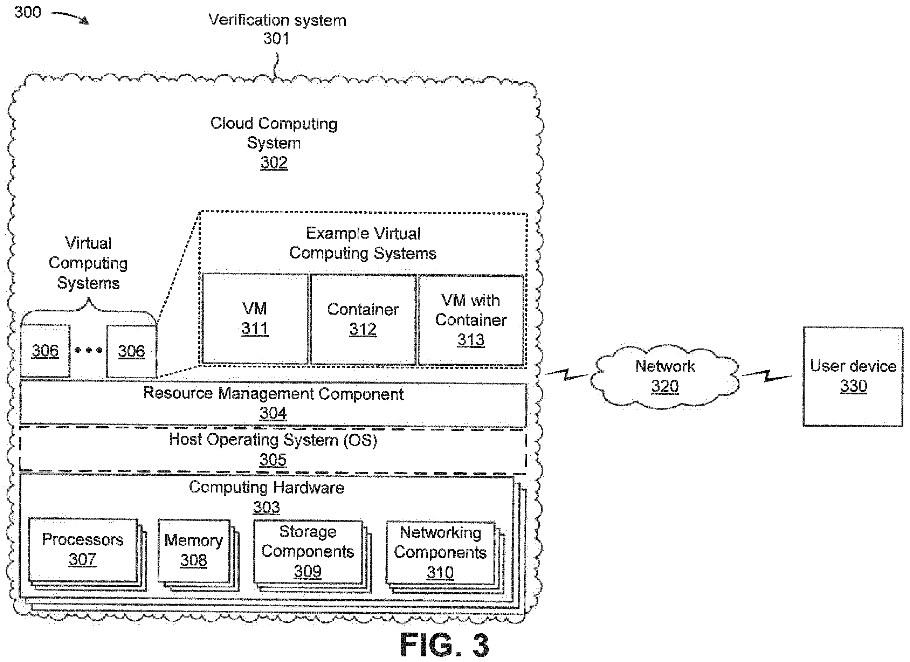

Fig. 3 is a diagram of an example environment 300 in which systems and/or methods described herein may be implemented. As shown in Fig. 3, environment 300 may include a verification system 301, which may include one or more elements of and/or may execute within a cloud computing system 302. The cloud computing system 302 may include one or more elements 303-313, as described in more detail below. As further shown in Fig. 3, environment 300 may include a network 320 and/or a user device 330. Devices and/or elements of environment 300 may interconnect via wired connections and/or wireless connections.

The cloud computing system 302 includes computing hardware 303, a resource management component 304, a host operating system (OS) 305, and/or one or more virtual computing systems 306. The resource management component 304 may perform virtualization (e.g., abstraction) of computing hardware 303 to create the one or more virtual computing systems 306. Using virtualization, the resource management component 304 enables a single computing device (e.g., a computer, a server, and/or the like) to operate like multiple computing devices, such as by creating multiple isolated virtual computing systems 306 from computing hardware 303 of the single computing device. In this way, computing hardware 303 can operate more efficiently, with lower power consumption, higher reliability, higher availability, higher utilization, greater flexibility, and lower cost than using separate computing devices.

Computing hardware 303 includes hardware and corresponding resources from one or more computing devices. For example, computing hardware 303 may include hardware from a single computing device (e.g., a single server) or from multiple computing devices (e.g., multiple servers), such as multiple computing devices in one or more data centers. As shown, computing hardware 303 may include one or more processors 307, one or more memories 308, one or more storage components 309, and/or one or more networking components 310. Examples of a processor, a memory, a storage component, and a networking component (e.g., a communication component) are described elsewhere herein.

The resource management component 304 includes a virtualization application (e.g., executing on hardware, such as computing hardware 303) capable of virtualizing computing hardware 303 to start, stop, and/or manage one or more virtual computing systems 306. For example, the resource management component 304 may include a hypervisor (e.g., a bare-metal or Type 1 hypervisor, a hosted or Type 2 hypervisor, and/or the like) or a virtual machine monitor, such as when the virtual computing systems 306 are virtual machines 311. Additionally, or alternatively, the resource management component 304 may include a container manager, such as when the virtual computing systems 306 are containers 312. In some implementations, the resource management component 304 executes within and/or in coordination with a host operating system 305.

A virtual computing system 306 includes a virtual environment that enables cloud-based execution of operations and/or processes described herein using computing hardware 303. As shown, a virtual computing system 306 may include a virtual machine 311, a container 312, a hybrid environment 313 that includes a virtual machine and a container, and/or the like. A virtual computing system 306 may execute one or more applications using a file system that includes binary files, software libraries, and/or other resources required to execute applications on a guest operating system (e.g., within the virtual computing system 306) or the host operating system 305.

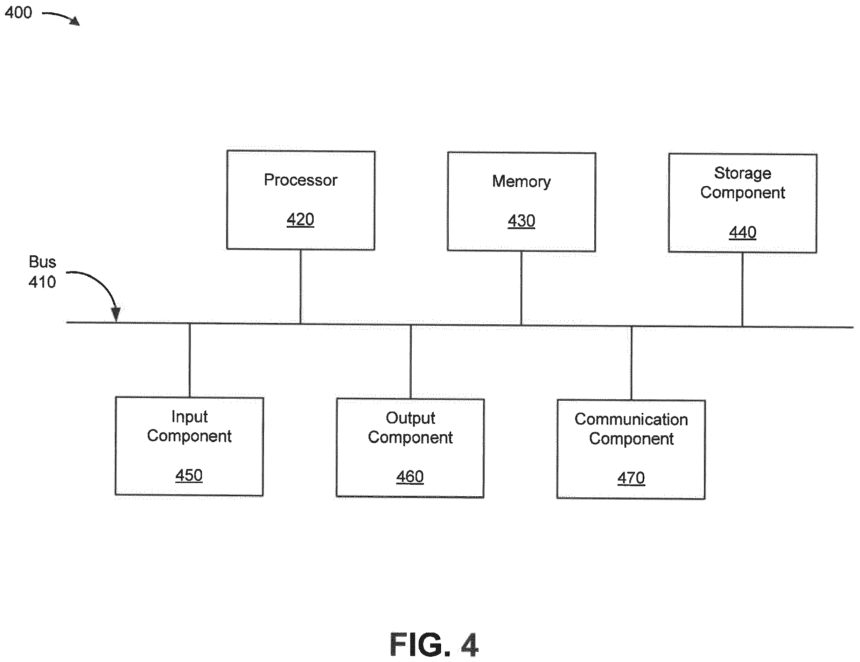

Although the verification system 301 may include one or more elements 303-313 of the cloud computing system 302, may execute within the cloud computing system 302, and/or may be hosted within the cloud computing system 302, in some implementations, the verification system 301 may not be cloud-based (e.g., may be implemented outside of a cloud computing system) or may be partially cloud-based. For example, the verification system 301 may include one or more devices that are not part of the cloud computing system 302, such as device 400 of Fig. 4, which may include a standalone server or another type of computing device. The verification system 301 may perform one or more operations and/or processes described in more detail elsewhere herein.

Network 320 includes one or more wired and/or wireless networks. For example, network 320 may include a cellular network, a public land mobile network (PLMN), a local area network (LAN), a wide area network (WAN), a private network, the Internet, and/or the like, and/or a combination of these or other types of networks. The network 320 enables communication among the devices of environment 300.

User device 330 includes one or more devices capable of receiving, generating, storing, processing, and/or providing information, as described elsewhere herein. User device 330 may include a communication device and/or a computing device. For example, user device 330 may include a wireless communication device, a user equipment (UE), a mobile phone (e.g., a smart phone or a cell phone, among other examples), a laptop computer, a tablet computer, a handheld computer, a desktop computer, a gaming device, a wearable communication device (e.g., a smart wristwatch or a pair of smart eyeglasses, among other examples), an Internet of Things (IoT) device, or a similar type of device. User device 330 may communicate with one or more other devices of environment 300, as described elsewhere herein.

The number and arrangement of devices and networks shown in Fig. 3 are provided as an example. In practice, there may be additional devices and/or networks, fewer devices and/or networks, different devices and/or networks, or differently arranged devices and/or networks than those shown in Fig. 3. Furthermore, two or more devices shown in Fig. 3 may be implemented within a single device, or a single device shown in Fig. 3 may be implemented as multiple, distributed devices. Additionally, or alternatively, a set of devices (e.g., one or more devices) of environment 300 may perform one or more functions described as being performed by another set of devices of environment 300.

Fig. 4 is a diagram of example components of a device 400, which may correspond to verification system 301 and/or user device 330. In some implementations, verification system 301 and/or user device 330 may include one or more devices 400 and/or one or more components of device 400. As shown in Fig. 4, device 400 may include a bus 410, a processor 420, a memory 430, a storage component 440, an input component 450, an output component 460, and a communication component 470.

Bus 410 includes a component that enables wired and/or wireless communication among the components of device 400. Processor 420 includes a central processing unit, a graphics processing unit, a microprocessor, a controller, a microcontroller, a digital signal processor, a field-programmable gate array, an application-specific integrated circuit, and/or another type of processing component. Processor 420 is implemented in hardware, firmware, or a combination of hardware and software. In some implementations, processor 420 includes one or more processors capable of being programmed to perform a function. Memory 430 includes a random-access memory, a read only memory, and/or another type of memory (e.g., a flash memory, a magnetic memory, and/or an optical memory).

Storage component 440 stores information and/or software related to the operation of device 400. For example, storage component 440 may include a hard disk drive, a magnetic disk drive, an optical disk drive, a solid-state disk drive, a compact disc, a digital versatile disc, and/or another type of non-transitory computer-readable medium. Input component 450 enables device 400 to receive input, such as user input and/or sensed inputs. For example, input component 450 may include a touch screen, a keyboard, a keypad, a mouse, a button, a microphone, a switch, a sensor, a global positioning system component, an accelerometer, a gyroscope, an actuator, and/or the like. Output component 460 enables device 400 to provide output, such as via a display, a speaker, and/or one or more light-emitting diodes. Communication component 470 enables device 400 to communicate with other devices, such as via a wired connection and/or a wireless connection. For example, communication component 470 may include a receiver, a transmitter, a transceiver, a modem, a network interface card, an antenna, and/or the like.

Device 400 may perform one or more processes described herein. For example, a non-transitory computer-readable medium (e.g., memory 430 and/or storage component 440) may store a set of instructions (e.g., one or more instructions, code, software code, program code, and/or the like) for execution by processor 420. Processor 420 may execute the set of instructions to perform one or more processes described herein. In some implementations, execution of the set of instructions, by one or more processors 420, causes the one or more processors 420 and/or the device 400 to perform one or more processes described herein. In some implementations, hardwired circuitry may be used instead of or in combination with the instructions to perform one or more processes described herein. Thus, implementations described herein are not limited to any specific combination of hardware circuitry and software.

The number and arrangement of components shown in Fig. 4 are provided as an example. Device 400 may include additional components, fewer components, different components, or differently arranged components than those shown in Fig. 4. Additionally, or alternatively, a set of components (e.g., one or more components) of device 400 may perform one or more functions described as being performed by another set of components of device 400.

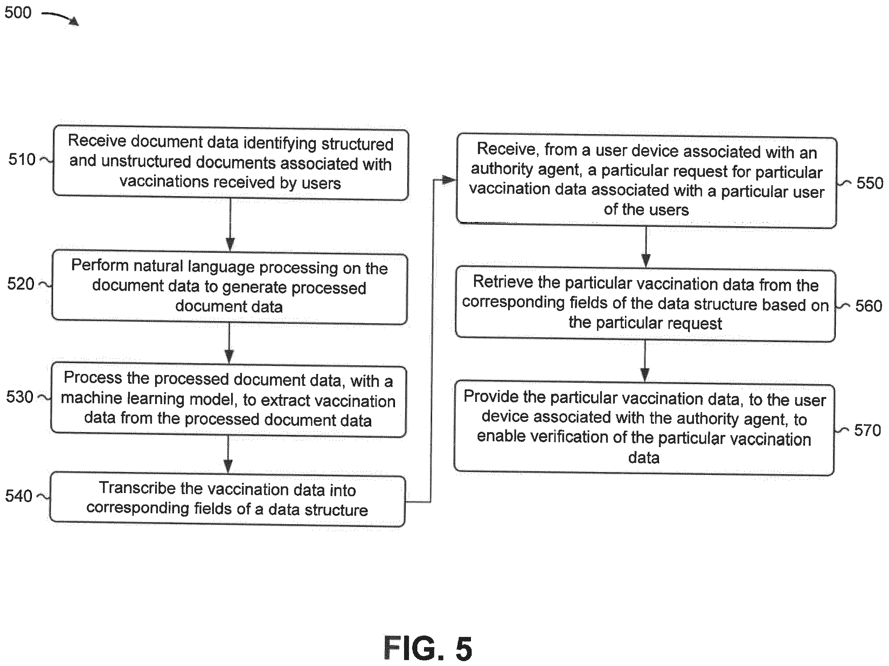

Fig. 5 is a flowchart of an example process 500 for utilizing machine learning and natural language processing to extract and verify vaccination data. In some implementations, one or more process blocks of Fig. 5 may be performed by a device (e.g., verification system 301). In some implementations, one or more process blocks of Fig. 5 may be performed by another device or a group of devices separate from or including the device, such as a user device (e.g., user device 330). Additionally, or alternatively, one or more process blocks of Fig. 5 may be performed by one or more components of device 400, such as processor 420, memory 430, storage component 440, input component 450, output component 460, and/or communication component 470.

As shown in Fig. 5, process 500 may include receiving, based on the request, document data identifying structured and unstructured documents associated with vaccinations received by users (block 510). For example, the device may receive, based on the request, document data identifying structured and unstructured documents associated with vaccinations received by users, as described above.

As further shown in Fig. 5, process 500 may include performing natural language processing on the document data to generate processed document data (block 520). For example, the device may perform natural language processing on the document data to generate processed document data, as described above.

As further shown in Fig. 5, process 500 may include processing the processed document data, with a machine learning model, to extract vaccination data from the processed document data (block 530). For example, the device may process the processed document data, with a machine learning model, to extract vaccination data from the processed document data, as described above.

As further shown in Fig. 5, process 500 may include transcribing the vaccination data into corresponding fields of a data structure (block 540). For example, the device may transcribe the vaccination data into corresponding fields of a data structure, as described above.

As further shown in Fig. 5, process 500 may include receiving, from a user device associated with an authority agent, a particular request for particular vaccination data associated with a user of the users (block 550). For example, the device may receive, from a user device associated with an authority agent, a particular request for particular vaccination data associated with a user of the users, as described above.

As further shown in Fig. 5, process 500 may include retrieving the particular vaccination data from the corresponding fields of the data structure based on the particular request (block 560). For example, the device may retrieve the particular vaccination data from the corresponding fields of the data structure based on the particular request, as described above.

As further shown in Fig. 5, process 500 may include providing the particular vaccination data, to the user device associated with the authority agent, to enable verification of the particular vaccination data (block 570). For example, the device may provide the particular vaccination data, to the user device associated with the authority agent, to enable verification of the particular vaccination data, as described above.

Process 500 may include additional implementations, such as any single implementation or any combination of implementations described below and/or in connection with one or more other processes described elsewhere herein.

In a first implementation, process 500 includes training, prior to receiving the document data, the machine learning model with historical document data identifying historical structured and unstructured documents associated with historical vaccinations.

In a second implementation, alone or in combination with the first implementation, the structured documents include embedded codes that enable arranging of information in specified formats, and the unstructured documents include free form arrangements in which structures, styles, and content of information from original documents are not preserved.

In a third implementation, alone or in combination with one or more of the first and second implementations, process 500 includes processing the document data with a computer vision model or with optical character recognition to generate homogeneous documents with a common format, and performing the natural language processing on the document data to generate the processed document data includes performing the natural language processing on the homogeneous documents to generate the processed document data.

In a fourth implementation, alone or in combination with one or more of the first through third implementations, process 500 includes determining that the particular request satisfies an access control requirement to access the particular vaccination data.

In a fifth implementation, alone or in combination with one or more of the first through fourth implementations, process 500 includes verifying the vaccination data, from the corresponding fields, with a registration authority.

In a sixth implementation, alone or in combination with one or more of the first through fifth implementations, processing the processed document data, with the machine learning model, to extract the vaccination data from the processed document data includes classifying the processed document data into categories and extracting the vaccination data from the processed document data based on the categories.

In a seventh implementation, alone or in combination with one or more of the first through sixth implementations, the structured documents include specified formats, the unstructured documents include a plurality of different formats, and process 500 includes transforming the specified formats of the structured documents, and the plurality of different formats of the unstructured documents, into a common format prior to performing the natural language processing on the document data.

In an eighth implementation, alone or in combination with one or more of the first through eighth implementations, processing the processed document data, with the machine learning model, to extract the vaccination data from the processed document data includes identifying one or more differences in the processed document data, receiving feedback associated with the one or more differences, and extracting the vaccination data from the processed document data based on the feedback.

In a ninth implementation, alone or in combination with one or more of the first through eighth implementations, verifying the vaccination data, from the corresponding fields, with the registration authority includes receiving, from the registration authority, feedback identifying one or more differences in the vaccination data, correcting the one or more differences identified in the feedback to generate corrected vaccination data, and verifying the corrected vaccination data with the registration authority.

In a tenth implementation, alone or in combination with one or more of the first through ninth implementations, process 500 includes receiving, from the user device associated with the authority agent, an additional information request associated with the particular vaccination data, identifying additional information based on the additional information request, and providing the additional information, to the user device associated with the authority agent, to enable verification of the particular vaccination data.

In an eleventh implementation, alone or in combination with one or more of the first through tenth implementations, process 500 includes receiving an update to the particular vaccination data associated with the user, and updating the particular vaccination data in the data structure based on the update.

In a twelfth implementation, alone or in combination with one or more of the first through eleventh implementations, the machine learning model includes a machine learning-based domain model associated with domain-specific terminology.

Although Fig. 5 shows example blocks of process 500, in some implementations, process 500 may include additional blocks, fewer blocks, different blocks, or differently arranged blocks than those depicted in Fig. 5. Additionally, or alternatively, two or more of the blocks of process 500 may be performed in parallel.

The foregoing disclosure provides illustration and description but is not intended to be exhaustive or to limit the implementations to the precise form disclosed. Modifications may be made in light of the above disclosure or may be acquired from practice of the implementations.

As used herein, the term "component" is intended to be broadly construed as hardware, firmware, or a combination of hardware and software. It will be apparent that systems and/or methods described herein may be implemented in different forms of hardware, firmware, and/or a combination of hardware and software. The actual specialized control hardware or software code used to implement these systems and/or methods is not limiting of the implementations. Thus, the operation and behavior of the systems and/or methods are described herein without reference to specific software code - it being understood that software and hardware can be used to implement the systems and/or methods based on the description herein.

As used herein, satisfying a threshold may, depending on the context, refer to a value being greater than the threshold, greater than or equal to the threshold, less than the threshold, less than or equal to the threshold, equal to the threshold, and/or the like, depending on the context.

Although particular combinations of features are recited in the claims and/or disclosed in the specification, these combinations are not intended to limit the disclosure of various implementations. In fact, many of these features may be combined in ways not specifically recited in the claims and/or disclosed in the specification. Although each dependent claim listed below may directly depend on only one claim, the disclosure of various implementations includes each dependent claim in combination with every other claim in the claim set.

No element, act, or instruction used herein should be construed as critical or essential unless explicitly described as such. Also, as used herein, the articles "a" and "an" are intended to include one or more items and may be used interchangeably with "one or more." Further, as used herein, the article "the" is intended to include one or more items referenced in connection with the article "the" and may be used interchangeably with "the one or more." Furthermore, as used herein, the term "set" is intended to include one or more items (e.g., related items, unrelated items, a combination of related and unrelated items, and/or the like), and may be used interchangeably with "one or more." Where only one item is intended, the phrase "only one" or similar language is used. Also, as used herein, the terms "has," "have," "having," or the like are intended to be open-ended terms. Further, the phrase "based on" is intended to mean "based, at least in part, on" unless explicitly stated otherwise. Also, as used herein, the term "or" is intended to be inclusive when used in a series and may be used interchangeably with "and/or," unless explicitly stated otherwise (e.g., if used in combination with "either" or "only one of').

In view of the above, the present application may disclose the following items:
Item 1. A method, comprising:
   receiving, by a device, document data identifying structured and unstructured documents associated with vaccinations received by users;
   performing, by the device, natural language processing on the document data to generate processed document data;
   processing, by the device, the processed document data, with a machine learning model, to extract vaccination data from the processed document data;
   transcribing, by the device, the vaccination data into corresponding fields of a data structure;
   receiving, by the device and from a user device associated with an authority agent, a particular request for particular vaccination data associated with a user of the users;
   retrieving, by the device, the particular vaccination data from the corresponding fields of the data structure based on the particular request; and
   providing, by the device, the particular vaccination data, to the user device associated with the authority agent, to enable verification of the particular vaccination data.
Item 2. The method of item 1, further comprising:
   training, prior to receiving the document data, the machine learning model with historical document data identifying historical structured and unstructured documents associated with historical vaccinations.
Item 3. The method of item 1 or 2, wherein the structured documents include embedded codes that enable arranging of information in specified formats, and
   wherein the unstructured documents include free form arrangements in which structures, styles, and content of information from original documents are not preserved.
Item 4. The method of any one of the preceding items, further comprising:
   processing the document data with a computer vision model or with optical character recognition to generate homogeneous documents with a common format,
   wherein performing the natural language processing on the document data to generate the processed document data comprises:
   performing the natural language processing on the homogeneous documents to generate the processed document data.
Item 5. The method of any one of the preceding items, further comprising:
   determining that the particular request satisfies an access control requirement to access the particular vaccination data.
Item 6. The method of any one of the preceding items, further comprising:
   verifying the vaccination data, from the corresponding fields, with a registration authority.
Item 7. The method of any one of the preceding items, wherein processing the processed document data, with the machine learning model, to extract the vaccination data from the processed document data comprises:
   classifying the processed document data into categories; and
   extracting the vaccination data from the processed document data based on the categories.
Item 8. A device, comprising:
   one or more memories; and
   one or more processors, coupled to the one or more memories, configured to:
      train a machine learning model with historical document data identifying historical structured and unstructured documents associated with historical vaccinations;
      provide a request for document data;
      receive, based on the request, document data identifying structured and unstructured documents associated with vaccinations received by users;
      perform natural language processing on the document data to generate processed document data;
      process the processed document data, with the machine learning model, to extract vaccination data from the processed document data;
      assign the vaccination data into corresponding fields of a data structure;
      verify the vaccination data, from the corresponding fields, with a registration authority;
      receive, from a user device associated with an authority agent, a particular request for particular vaccination data associated with a user of the users;
      retrieve the particular vaccination data from the corresponding fields of the data structure based on the particular request; and
      provide the particular vaccination data, to the user device associated with the authority agent, to enable verification of the particular vaccination data.
Item 9. The device of item 8, wherein the structured documents include specified formats, the unstructured documents include a plurality of different formats, and the one or more processors are further configured to:
   transform the specified formats of the structured documents, and the plurality of different formats of the unstructured documents, into a common format prior to performing the natural language processing on the document data.
Item 10. The device of item 8 or 9, wherein, to process the processed document data, with the machine learning model, to extract the vaccination data from the processed document data, the one or more processors are configured to:
   identify one or more differences in the processed document data;
   receive feedback associated with the one or more differences; and extract the vaccination data from the processed document data based on the feedback.
Item 11. The device of any one of items 8 to 10, wherein, to verify the vaccination data, from the corresponding fields, with the registration authority, the one or more processors are configured to:
   receive, from the registration authority, feedback identifying one or more differences in the vaccination data;
   correct the one or more differences identified in the feedback to generate corrected vaccination data; and
   verify the corrected vaccination data with the registration authority.
Item 12. The device of any one of items 8 to 11, wherein the one or more processors are further configured to:
   receive, from the user device associated with the authority agent, an additional information request associated with the particular vaccination data;
   identify additional information based on the additional information request; and
   provide the additional information, to the user device associated with the authority agent, to enable verification of the particular vaccination data.
Item 13. The device of any one of items 8 to 12, wherein the one or more processors are further configured to:
   receive an update to the particular vaccination data associated with the user; and
   update the particular vaccination data in the data structure based on the update.
Item 14. The device of any one of items 8 to 13, wherein the machine learning model includes a machine learning-based domain model associated with domain-specific terminology.
Item 15. A non-transitory computer-readable medium storing a set of instructions, the set of instructions comprising:
   one or more instructions that, when executed by one or more processors of a device, cause the device to:
   provide a request for document data;
   receive, based on the request, document data identifying structured and unstructured documents associated with vaccinations received by users;
   perform natural language processing on the document data to generate processed document data;
   process the processed document data, with a machine learning model, to extract vaccination data from the processed document data;
   transcribe the vaccination data into corresponding fields of a data structure; and
   verify the vaccination data, from the corresponding fields, with a registration authority.
Item 16. The non-transitory computer-readable medium of item 15, wherein the one or more instructions further cause the device to:
   receive, from a user device associated with an authority agent, a particular request for particular vaccination data associated with a user of the users;
   retrieve the particular vaccination data from the corresponding fields of the data structure based on the particular request; and
   provide the particular vaccination data, to the user device associated with the authority agent, to enable verification of the particular vaccination data.
Item 17. The non-transitory computer-readable medium of item 15 or 16, wherein the one or more instructions further cause the device to:
   process the document data with a computer vision model or with optical character recognition to generate homogeneous documents with a common format,
   wherein the one or more instructions, that cause the device to perform the natural language processing on the document data to generate the processed document data, cause the device to:
   perform the natural language processing on the homogeneous documents to generate the processed document data.
Item 18. The non-transitory computer-readable medium of any one of items 15 to 17, wherein the one or more instructions, that cause the device to process the processed document data, with the machine learning model, to extract the vaccination data from the processed document data, cause the device to:
   classify the processed document data into categories; and
   extract the vaccination data from the processed document data based on the categories.
Item 19. The non-transitory computer-readable medium of any one of items 15 to 18, wherein the one or more instructions, that cause the device to process the processed document data, with the machine learning model, to extract the vaccination data from the processed document data, cause the device to:
   identify one or more differences in the processed document data;
   receive feedback associated with the one or more differences; and extract the vaccination data from the processed document data based on the feedback.
Item 20. The non-transitory computer-readable medium of any one of items 15 to 19, wherein the one or more instructions, that cause the device to verify the vaccination data, from the corresponding fields, with the registration authority, cause the device to:
   receive, from the registration authority, feedback identifying one or more differences in the vaccination data;
   correct the one or more differences identified in the feedback to generate corrected vaccination data; and
   verify the corrected vaccination data with the registration authority.

## Claims

1. A method, comprising:
receiving, by a device, document data identifying structured and unstructured documents associated with vaccinations received by users;
performing, by the device, natural language processing on the document data to generate processed document data;
processing, by the device, the processed document data, with a machine learning model, to extract vaccination data from the processed document data;
transcribing, by the device, the vaccination data into corresponding fields of a data structure;
receiving, by the device and from a user device associated with an authority agent, a particular request for particular vaccination data associated with a user of the users;
retrieving, by the device, the particular vaccination data from the corresponding fields of the data structure based on the particular request; and
providing, by the device, the particular vaccination data, to the user device associated with the authority agent, to enable verification of the particular vaccination data.

2. The method of claim 1, further comprising:
training, prior to receiving the document data, the machine learning model with historical document data identifying historical structured and unstructured documents associated with historical vaccinations.

3. The method of claim 1 or 2, wherein the structured documents include embedded codes that enable arranging of information in specified formats, and
wherein the unstructured documents include free form arrangements in which structures, styles, and content of information from original documents are not preserved.

4. The method of any one of the preceding claims, further comprising:
processing the document data with a computer vision model or with optical character recognition to generate homogeneous documents with a common format,
wherein performing the natural language processing on the document data to generate the processed document data comprises:
performing the natural language processing on the homogeneous documents to generate the processed document data;
and/or
further comprising:
determining that the particular request satisfies an access control requirement to access the particular vaccination data;
and/or
further comprising:
verifying the vaccination data, from the corresponding fields, with a registration authority.

5. The method of any one of the preceding claims, wherein processing the processed document data, with the machine learning model, to extract the vaccination data from the processed document data comprises:
classifying the processed document data into categories; and
extracting the vaccination data from the processed document data based on the categories.

6. A device, comprising:
one or more memories; and
one or more processors, coupled to the one or more memories, configured to:
train a machine learning model with historical document data identifying historical structured and unstructured documents associated with historical vaccinations;
provide a request for document data;
receive, based on the request, document data identifying structured and unstructured documents associated with vaccinations received by users;
perform natural language processing on the document data to generate processed document data;
process the processed document data, with the machine learning model, to extract vaccination data from the processed document data;
assign the vaccination data into corresponding fields of a data structure;
verify the vaccination data, from the corresponding fields, with a registration authority;
receive, from a user device associated with an authority agent, a particular request for particular vaccination data associated with a user of the users;
retrieve the particular vaccination data from the corresponding fields of the data structure based on the particular request; and
provide the particular vaccination data, to the user device associated with the authority agent, to enable verification of the particular vaccination data.

7. The device of claim 6, wherein the structured documents include specified formats, the unstructured documents include a plurality of different formats, and the one or more processors are further configured to:
transform the specified formats of the structured documents, and the plurality of different formats of the unstructured documents, into a common format prior to performing the natural language processing on the document data.

8. The device of claim 6 or 7, wherein, to process the processed document data, with the machine learning model, to extract the vaccination data from the processed document data, the one or more processors are configured to:
identify one or more differences in the processed document data;
receive feedback associated with the one or more differences; and
extract the vaccination data from the processed document data based on the feedback.

9. The device of any one of claims 6 to 8, wherein, to verify the vaccination data, from the corresponding fields, with the registration authority, the one or more processors are configured to:
receive, from the registration authority, feedback identifying one or more differences in the vaccination data;
correct the one or more differences identified in the feedback to generate corrected vaccination data; and
verify the corrected vaccination data with the registration authority.

10. The device of any one of claims 6 to 9, wherein the one or more processors are further configured to:
receive, from the user device associated with the authority agent, an additional information request associated with the particular vaccination data;
identify additional information based on the additional information request; and
provide the additional information, to the user device associated with the authority agent, to enable verification of the particular vaccination data;
and/or
wherein the one or more processors are further configured to:
receive an update to the particular vaccination data associated with the user; and
update the particular vaccination data in the data structure based on the update;
and/or
wherein the machine learning model includes a machine learning-based domain model associated with domain-specific terminology.

11. A non-transitory computer-readable medium storing a set of instructions, the set of instructions comprising:
one or more instructions that, when executed by one or more processors of a device, cause the device to:
provide a request for document data;
receive, based on the request, document data identifying structured and unstructured documents associated with vaccinations received by users;
perform natural language processing on the document data to generate processed document data;
process the processed document data, with a machine learning model, to extract vaccination data from the processed document data;
transcribe the vaccination data into corresponding fields of a data structure; and
verify the vaccination data, from the corresponding fields, with a registration authority.

12. The non-transitory computer-readable medium of claim 11, wherein the one or more instructions further cause the device to:
receive, from a user device associated with an authority agent, a particular request for particular vaccination data associated with a user of the users;
retrieve the particular vaccination data from the corresponding fields of the data structure based on the particular request; and
provide the particular vaccination data, to the user device associated with the authority agent, to enable verification of the particular vaccination data;
and/or
wherein the one or more instructions further cause the device to:
process the document data with a computer vision model or with optical character recognition to generate homogeneous documents with a common format,
wherein the one or more instructions, that cause the device to perform the natural language processing on the document data to generate the processed document data, cause the device to:
perform the natural language processing on the homogeneous documents to generate the processed document data.

13. The non-transitory computer-readable medium of claim 11 or 12, wherein the one or more instructions, that cause the device to process the processed document data, with the machine learning model, to extract the vaccination data from the processed document data, cause the device to:
classify the processed document data into categories; and
extract the vaccination data from the processed document data based on the categories.

14. The non-transitory computer-readable medium of any one of claims 11 to 13, wherein the one or more instructions, that cause the device to process the processed document data, with the machine learning model, to extract the vaccination data from the processed document data, cause the device to:
identify one or more differences in the processed document data;
receive feedback associated with the one or more differences; and
extract the vaccination data from the processed document data based on the feedback.

15. The non-transitory computer-readable medium of any one of claims 11 to 14, wherein the one or more instructions, that cause the device to verify the vaccination data, from the corresponding fields, with the registration authority, cause the device to:
receive, from the registration authority, feedback identifying one or more differences in the vaccination data;
correct the one or more differences identified in the feedback to generate corrected vaccination data; and
verify the corrected vaccination data with the registration authority.
